# EUROPÄISCHE PATENTSCHRIFT

(11) **EP 2 441 431 B1**
(45) Veröffentlichungstag und Bekanntmachung des Hinweises auf die Patenterteilung: **13.04.2016**
(21) Anmeldenummer: 11176691.1
(22) Anmeldetag: 05.08.2011
(51) Int. Cl.: A61J 1/20, A61J 1/06, A61J 1/14

(54) **Behälter für die Herstellung einer konditionierten Blutzusammensetzung**
Container for the production of a conditioned blood compound
Récipient pour la fabrication d'une composition sanguine conditionnée

(30) Priorität: 12.10.2010 DE 102010042356
(43) Veröffentlichungstag der Anmeldung: 18.04.2012
(73) Patentinhaber: Henke-Sass, Wolf GmbH, 78532 Tuttlingen (DE)
(72) Erfinder: Rückert, Ralf, 78573 Wurmlingen (DE); Müller, Fabian, 78573 Wurmlingen (DE)
(74) Vertreter: Patentanwälte Geyer, Fehners & Partner mbB

(56) Entgegenhaltungen:
- DE-A1-102006 005 016
- DE-A1-102008 023 545
- US-A- 3 630 199

## Beschreibung

Die vorliegende Erfindung betrifft einen Behälter für die Herstellung einer konditionierten Blutzusammensetzung, die insbesondere IL-1Ra enthält, mit den Merkmalen des Oberbegriffs des Anspruches 1.

Ein solcher Behälter ist z.B. aus der DE 10 2008 023 545 A1 bekannt.

Ein Verfahren zur Herstellung von IL-1 Ra in einer Spritze ist beispielsweise aus der EP 1 151 004 B1 bekannt. Dabei wird einem Patienten (einem Menschen oder einem Tier) mittels einer Spritze Blut entnommen, wobei die Spritze Glaskugeln enthalten kann. Die Spritze mit dem entnommenen Blut wird dann inkubiert, wobei die Inkubation bevorzugt über einen Zeitraum von 12-72 Stunden und vorzugsweise bei einer Temperatur von 20 °C - 41 °C durchgeführt wird. Danach kann die Spritze mit dem darin enthaltenen Blut zentrifugiert werden und das Blutplasma wird mittels einer weiteren Spritze zur weiteren Verwendung entnommen.

Obwohl bis zur Entnahme des Blutplasmas kein Umfüllen des Blutes notwendig ist, ist die Handhabung dennoch aufwendig. Insbesondere muß in der Regel für das Zentrifugieren der aus dem Spritzenzylinder überstehende Teil der Kolbenstange abgebrochen werden. Auch ist ein Verschließen des vorderen Endes der Spritze notwendig, wozu die Spritzenkanüle entfernt und statt dessen ein Verschlußstopfen vorzusehen ist.

Ausgehend hiervon ist es Aufgabe der Erfindung, einen Behälter der eingangs genannten Art für die Herstellung einer konditionierten Blutzusammensetzung so weiterzubilden, dass eine vereinfachte Handhabung insbesondere bei einem Zentrifugierschritt möglich ist.

Erfindungsgemäß wird die Aufgabe bei einem Behälter der eingangs genannten Art dadurch gelöst, dass die Abmessungen der Einfüllöffnung so gewählt sind, dass die Teilchen zu groß für die Einfüllöffnung sind und somit nicht durch sie hindurchpassen und dass, wenn das Abgabeende der Spritze zum Einfüllen des Blutes in der Einfüllöffnung positioniert ist, ein Entlüftungsspalt zwischen dem Abgabeende und der Innenseite der Einfüllöffnung vorhanden ist.

Der Entlüftungsspalt liegt insbesondere auch vor, wenn das Abgabeende der Spritze zum Einfüllen des Blutes in der Einfüllöffnung über die gesamte Länge der Einfüllöffnung oder sogar darüber hinaus bis in den Hohlraum positioniert ist.

Aufgrund des erfindungsgemäßen Behälters muß lediglich das entnommene Blut in den Behälter eingefüllt werden, was aufgrund des beim Einfüllen vorhandenen Ent- bzw. Belüftungsspaltes ohne Probleme möglich ist. Danach kann der Behälter zusammen mit dem eingefüllten Blut zentrifugiert werden. Somit ist eine einfache Handhabung möglich. Insbesondere müssen keine Kolbenstangen abgebrochen werden und Kanülen durch Verschlußstopfen ersetzt werden.

Die Einfüllöffnung dient hier somit insbesondere zum Druckausgleich zwischen dem Hohlraum und dem Äußeren (bzw. der Umgebung) und zwar unabhängig davon, ob das Abgabeende in der Einfüllöffnung positioniert ist oder nicht. Aufgrund der Einfüllöffnung ist der Behälter für die einzufüllende Flüssigkeit (z.B. Blut) offen und nicht dicht verschlossen. Für die Teilchen ist der Behälter aufgrund der Abmessungen der Einfüllöffnung jedoch verschlossen bzw. dicht.

Die Einfüllöffnung ist insbesondere als offenes Durchgangsloch bzw. als offene Durchgangsbohrung ausgebildet.

Durch die erfindungsgemäße Konstruktion des Behälters und insbesondere der Verschlußkappe müssen z.B. keine Entlüftungsventile in der Verschlußkappe vorgesehen werden, wodurch die Verschlußkappe kostengünstig z.B. als Spritzgußteil hergestellt werden kann. Insbesondere ist die Verschlußkappe bis auf die Einfüllöffnung frei von weiteren Be- bzw. Entlüftungselementen, wie z.B. Ventilen. Auch die Wandung ist bevorzugt frei von weiteren Be- bzw. Entlüftungselementen.

Unter einer konditionierten Blutzusammensetzung wird hier insbesondere die Blutzusammensetzung verstanden, die man nach dem Zentrifugieren des Behälters mit dem darin enthaltenen Blut erhält. Insbesondere kann es sich um das nach dem Zentrifugieren vorliegende Blutplasma oder -serum handeln.

Die im Behälter enthaltenen Teilchen dienen im wesentlichen dazu, die dem eingefüllten Blut bereitgestellte Oberfläche zu vergrößern, was sich vorteilhaft bei der Herstellung der konditionierten Blutzusammensetzung erwiesen hat. Insbesondere die Herstellung von IL-1Ra (Interleukin 1-Rezeptor-Antagonist) kann dadurch angeregt oder unterstützt werden. Auch die gewünschte Selektivität im Vergleich zu IL-1β von größer als 10:1 und insbesondere von größer als 100:1 kann dadurch erreicht werden. Natürlich ist der Behälter auch dazu geeignet, zusätzliche oder andere Cytokine, die bevorzugt therapeutisch wirksam sind, zu erzeugen und/oder anzureichern.

Bei dem erfindungsgemäßen Behälter kann die Wandung als starre Wandung ausgebildet sein. Insbesondere kann der Behälter im wesentlichen hohlzylinderförmig ausgebildet sein.

Ferner kann die Verschlußkappe die Öffnung so verschließen, daß sie nicht zerstörungsfrei entfernbar ist.

Die Verschlußkappe kann mittels einer Rastverbindung mit der Wandung verbunden sein. Es ist jedoch auch jede andere Art der Verbindung möglich. Ferner kann eine formschlüssige, kraftschlüssige und/oder stoffschlüssige Verbindung zwischen Verschlußkappe und Wandung vorliegen.

Die Wandung und die Verschlußkappe sind bevorzugt aus dem gleichen Material hergestellt. Insbesondere wird als Material Kunststoff eingesetzt, wie z.B. Polypropylen, Polystyrol, Polyvinylchlorid oder Polyethylen.

Natürlich ist es auch möglich, daß die Wandung und die Verschlußkappe aus unterschiedlichen Materialien hergestellt sind. Insbesondere kann die Wandung auch aus einem Glasmaterial hergestellt sein. Die Verschlußkappe ist bevorzugt dann aus einem Kunststoffmaterial hergestellt.

Bei dem erfindungsgemäßen Behälter kann die Einfüllöffnung Bestandteil eines Luer Lock Anschlusses sein. Damit ist es möglich, herkömmliche Verschlußstopfen einzusetzen, um den Behälter nach dem Befüllen mit dem Blut zu verschließen. Der so verschlossene Behälter kann dann zentrifugiert werden.

Die in dem Behälter angeordneten Teilchen können im wesentlichen kugelförmig ausgebildet sein. Es ist aber auch jede andere Raumform möglich. Bevorzugt sind die Teilchen gleich groß oder ist sichergestellt, daß sie eine Mindestgröße nicht unterschreiten. Basierend auf der Mindestgröße kann dann durch geeignete Wahl der Abmessungen der Einfüllöffnung sichergestellt werden, daß die Teilchen dauerhaft im Hohlraum eingesperrt sind.

Die Teilchen können aus Glas oder Kunststoff hergestellt sein. Insbesondere können Sie aus Bor- bzw. Borosilikatglas hergestellt sein.

In dem Behälter kann ein Antikoagulanzmittel enthalten sein, um die Gerinnung des eingefüllten Blutes zu verhindern. Es ist jedoch auch möglich, daß keinerlei Antikoagulanzmittel im Behälter enthalten ist.

Der erfindungsgemäße Behälter kann so ausgebildet sein, daß die Wandung nur die eine Öffnung aufweist.

Bei dem erfindungsgemäßen Behälter kann die Wandung einstückig ausgebildet sein.

Des weiteren ist der erfindungsgemäße Behälter insbesondere so ausgebildet, daß das Volumen des Hohlraums mit Ausnahme der eingefüllten Teilchen (z.B. Kugeln) und des einzufüllenden Blutes nicht veränderbar bzw. konstant ist. Der erfindungsgemäße Behälter ist also insbesondere keine Spritze, da bei einer Spritze der Hohlraum des Spritzenzylinders durch Bewegung des Kolbens veränderbar ist.

Der erfindungsgemäße Behälter ist insbesondere frei von bewegbaren Kolben oder einem sonstigen bewegbaren Element, mit dem durch Betätigung von außen das Volumen des Hohlraums wiederholt verkleinert und vergrößert werden kann.

Ferner kann der Behälter einen Verschlußstopen zum Verschließen der Einfüllöffnung aufweisen.

Es wird ferner ein System zur Herstellung einer konditionierten Blutzusammensetzung bereitgestellt, das einen erfindungsgemäßen Behälter (einschließlich der erfindungsgemäßen Weiterbildung) sowie eine ein Abgabeende aufweisende Spritze enthält. Das Abgabeende der Spritze ist dabei so ausgebildet, daß ein Entlüftungsspalt zwischen dem Abgabeende und der Innenseite der Einfüllöffnung vorhanden ist, wenn das Abgabeende der Spritze zum Einfüllen von in der Spritze enthaltenem Blut in der Einfüllöffnung positioniert ist (das Abgabeende kann insbesondere so positioniert sein, daß es über die gesamte Länge der Einfüllöffnung in dieser positioniert ist und ggf. darüber hinaus in den Hohlraum einsteht).

Das Abgabeende der Spritze kann als Kanüle ausgebildet sein, die mit der Spritze verbindbar ist. Insbesondere kann die Spritze als Einmalspritze und die Kanüle als Einmalkanüle ausgebildet sein.

Bevorzugt sind die einzelnen Elemente des Systems (Behälter, Spritze und ggf. Kanüle) einzeln steril verpackt.

Das System kann ferner noch den Verschlußstopfen zum Verschließen der Einfüllöffnung aufweisen. Auch der Verschlußstopfen ist bevorzugt einzeln steril verpackt.

Des weiteren kann das System mehrere Spritzen und/oder mehrere Kanülen aufweisen, die bevorzugt alle jeweils einzeln steril verpackt sind. Bei der Spritze bzw. den Spritzen sowie der Kanüle bzw. den Kanülen handelt es sich bevorzugt um Einwegartikel. Auch der erfindungsgemäße Behälter ist bevorzugt als Einwegartikel ausgebildet.

Es wird ferner bereitgestellt ein Verfahren zur Herstellung einer konditionierten Blutzusammensetzung, bei dem das Abgabeende einer Spritze, die Blut enthält, in der Einfüllöffnung eines erfindungsgemäßen Behälters so positioniert wird, daß der Entlüftungsspalt vorhanden ist, dann das Blut von der Spritze in den Hohlraum des Behälters eingefüllt wird und danach der Behälter mit dem Blut zentrifugiert wird, um die konditionierte Blutzusammensetzung herzustellen.

Der Schritt des Zentrifugierens wird bevorzugt bei Raumtemperatur durchgeführt. Insbesondere kann der Zentrifugierschritt bei einer Temperatur im Bereich von 20 °C - 41 °C durchgeführt werden. Die Dauer des Zentrifugierens kann z. B. 10, 15 oder 20 min betragen. Natürlich sind auch längere oder kürzere Zentrifugierzeiten möglich. Eine bevorzugte Zentrifugiergeschwindigkeit beträgt 3500 - 5500 Umdrehungen pro Minute, insbesondere 4500 Umdrehungen pro Minute.

Ferner kann noch ein Lagerungsschritt (bevorzugt vor dem Zentrifugieren) des Behälters mit dem eingefüllten Blut durchgeführt werden. Auch dieser Lagerungsschritt wird bevorzugt bei Raumtemperatur und insbesondere bei einer Temperatur im Bereich von 20 °C - 41 °C durchgeführt. Die Zeitdauer des Lagerungsschrittes kann vom Minutenbereich bis zum Stundenbereich betragen. So sind Lagerungszeiten von 10-30 min, 30-60 min oder im Bereich von 2 bis 72 Stunden möglich.

Bei dem erfindungsgemäßen Verfahren kann nach dem Zentrifugierschritt mittels einer Spritze zumindest Teile der im Behälter hergestellten konditionierten Blutzusammensetzung über die Einfüllöffnung entnommen werden. Die Spritze zum Entnehmen weist dabei wiederum ein Abgabeende auf, das so ausgebildet ist, daß ein Entlüftungsspalt vorhanden ist, wenn das Abgabeende in der Einfüllöffnung positioniert ist. Das Abgabeende kann insbesondere wiederum eine Kanüle sein.

Insbesondere wird das nach dem Zentrifugierschritt vorhandene Blutserum oder -plasma entnommen. Die entnommene konditionierte Blutzusammensetzung, die bevorzugt ein oder mehrere therapeutisch wirksame Cytokine (wie z.B. IL-1 Ra, IL 6, etc.) enthält, kann dann therapeutisch verwendet werden. Insbesondere kann sie demselben Patienten, dem das Blut entnommen wurde, wieder injiziert werden, z.B. an einer Nervenwurzel oder einem Gelenk.

Ferner kann das erfindungsgemäße Verfahren den Schritt der Blutentnahme aufweisen, wobei bevorzugt die Spritze, mit der das Blut entnommen wird, auch dazu genutzt wird, um das Blut über die Einfüllöffnung in den Behälter einzufüllen. Dabei kann die Spritze unverändert verwendet werden. Es ist jedoch auch möglich, daß die Kanüle zur Blutentnahme von der Spritze entfernt wird und dann eine weitere, sterile Kanüle mit der Spritze verbunden wird, um über diese Kanüle das Blut in den Behälter einzufüllen. Die weitere Kanüle kann auch weggelassen werden, wenn das Abgabeende der Spritze (ohne Kanüle) so ausgebildet ist, daß beim Positionieren des Abgabeendes in der Einfüllöffnung des Behälters der Entlüftungsspalt vorliegt.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine perspektivische Ansicht einer Ausführungsform des erfindungsgemäßen Behälters für die Herstellung einer konditionierten Blutzusammensetzung;
- Fig. 2: eine Schnittansicht des Behälters 1 von Fig. 1, wobei die eingefüllten Kugeln 8 nicht in Schnittdarstellung gezeigt sind;
- Fig. 3: eine perspektivische Ansicht der Verschlußkappe 9 des Behälters 1 von Fig. 1;
- Fig. 4: eine Schnittansicht der Verschlußkappe 9 von Fig. 3;
- Fig. 5: eine vergrößerte Ansicht des Details A von Fig. 2;
- Fig. 6: eine Schnittansicht des Verschlußstopfens 13;
- Fig. 7: eine Ansicht zur Erläuterung des Einfüllens des Blutes von der Spritze 22 in den Behälter 1, wobei die Spritze 22 in Seitenansicht und der Behälter 1 in einer Schnittansicht gemäß Fig. 2 dargestellt sind;
- Fig. 8: eine Seitenansicht der Spritze 22 von Fig. 7, und
- Fig. 9: eine Seitenansicht der Kanüle 21 von Fig. 7.

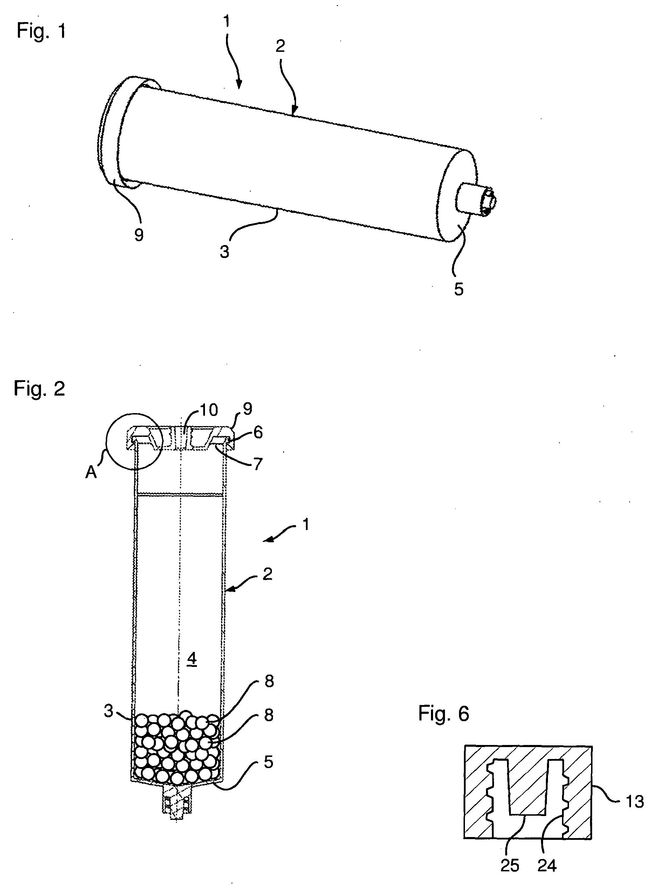

Bei der in Figuren 1 und 2 gezeigten Ausführungsform umfaßt der erfindungsgemäße Behälter 1 für die Herstellung einer konditionierten Blutzusammensetzung ein im wesentlichen hohlzylinderförmigen Hauptkörper 2 mit kreisförmigem Querschnitt, wobei die Wandung 3 des Hauptkörpers 2 einen Hohlraum 4 (Innenraum des Hauptkörpers 2) begrenzt.

Wie am besten aus Figur 2 ersichtlich ist, ist das untere Ende 5 des Hauptkörpers 2 verschlossen und das obere Ende 6 offen ausgebildet, so daß der Hauptkörper 2 genau eine Öffnung 7 (am oberen Ende 6) aufweist. Bei dem hier beschriebenen Ausführungsbeispiel ist der Hauptkörper 2 aus Polypropylen hergestellt.

Wie ferner Figur 2 zu entnehmen ist, sind eine Mehrzahl von Teilchen 8 in den Hohlraum 4 des Hauptkörpers 2 eingefüllt. Die Teilchen können beispielsweise kugelförmig und aus Borsilikatglas hergestellt sein.

Das Gesamtvolumen aller Kugeln 8 kann beispielsweise ca. 1/5 des Volumens des einzufüllenden Blutes aufweisen. Der in Figur 1 und 2 gezeigte Behälter 1 ist für 50 ml Blut ausgelegt, so daß das Gesamtvolumen der Teilchen 8 ca. 10 ml beträgt. Die Anzahl der Teilchen 8 ergibt sich dann in Abhängigkeit des Kugeldurchmessers. Natürlich kann das Gesamtvolumen aller Teilchen 8 im Verhältnis zu dem Volumen des einzufüllenden Blutes auch größer oder kleiner als 1/5 sein.

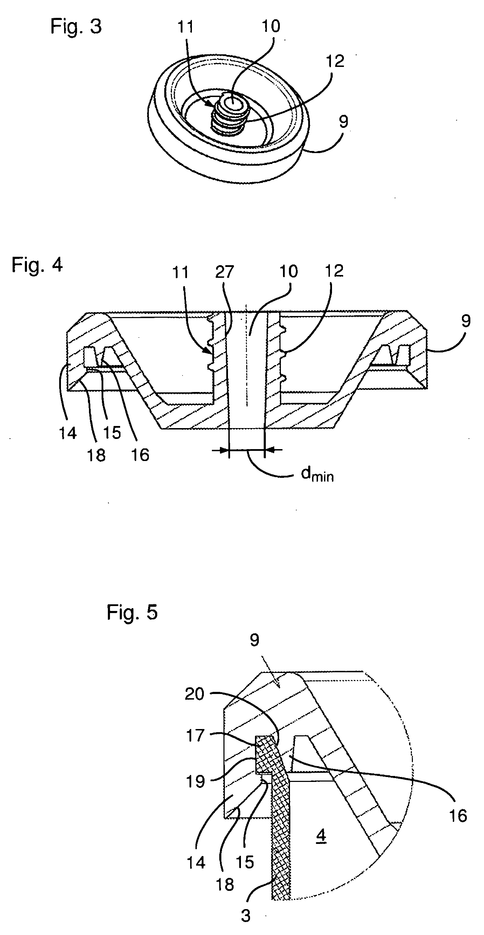

Die Öffnung 7 des Hauptkörpers 2 ist mit einer Verschlußkappe 9 verschlossen, der eine Einfüllöffnung 10 aufweist. Die Einfüllöffnung 10 ist ein Durchgangsloch und Bestandteil eines weiblichen Luer Lock Anschlusses 11, wie insbesondere den Darstellungen in Figuren 3 und 4 zu entnehmen ist.

Die Einfüllöffnung 10 weist einen kreisförmigen Querschnitt auf und ist konisch ausgebildet, wobei der Durchmesser in Richtung vom oberen zum unteren Ende 6,5 hin abnimmt.

Der Durchmesser der Kugeln 8 ist so gewählt, daß er größer ist als der minimale Durchmesser dₘᵢₙ der Einfüllöffnung 10 (Fig. 4), so daß die Kugeln 8 nicht durch die Einfüllöffnung 10 hindurchpassen und somit sichergestellt ist, daß die Kugeln 8 im durch die Verschlußkappe 9 verschlossenen Hohlraum 4 verbleiben.

Ferner weist der Luer Lock Anschluß 11 noch in üblicher Weise ein Außengewinde 12 für einen Verschlußstopfen 13 (Fig. 6) auf.

Die Verschlußkappe 9 ist mittels Form- und Kraftschluß mit dem oberen Ende 6 des Hauptkörpers 2 fest verbunden. Dazu weist die Verschlußkappe 9 einen Außenring 14 auf (Fig. 4), der sich im mit dem Hauptkörper 2 verbundenen Zustand in Längsrichtung des Hauptkörpers 2 erstreckt und einen radial nach innen vorstehenden Rastring 15 aufweist. Ferner weist die Verschlußkappe 9 noch eine sich in Längsrichtung erstreckende ringförmige Dichtlippe 16 auf.

Wie der vergrößerten Ansicht des Details A von Figur 2 in Figur 5 zu entnehmen ist, umgreift der Außenring 14 zusammen mit dem Rastring 15 einen am oberen Ende 6 ausgebildeten, ringförmigen Befestigungsabschnitt 17 der Wandung 3, so daß ein sicherer Sitz der Verschlußkappe 9 am Hauptkörper 2 erreicht wird. Um diesen Befestigungszustand zu erreichen, wird die Verschlußkappe 9 von oben auf das obere Ende 6 geschoben, wobei dabei aufgrund des angefasten Abschnitts 18 des Außenrings 14 dieser radial nach außen gedrückt wird, was aufgrund der Elastizität des Materials möglich ist. Sobald die Verschlußkappe 9 soweit über den Befestigungsabschnitt 17 geschoben wurde, daß der Rastring 15 nicht mehr an der nach außen weisenden Seite 19 des Befestigungsabschnitts 17 anliegt, gelangt man aufgrund der Elastizität des Außenrings 14 zu dem in Fig. 5 gezeigten Zustand. Gleichzeitig liegt die Dichtlippe 16 an der nach innen weisenden Seite 20 des Befestigungsabschnitts 17 an, so daß am oberen Ende 6 des Hauptkörpers die gewünschte Abdichtung des Hohlraums 4 gegenüber dem Äußeren vorliegt.

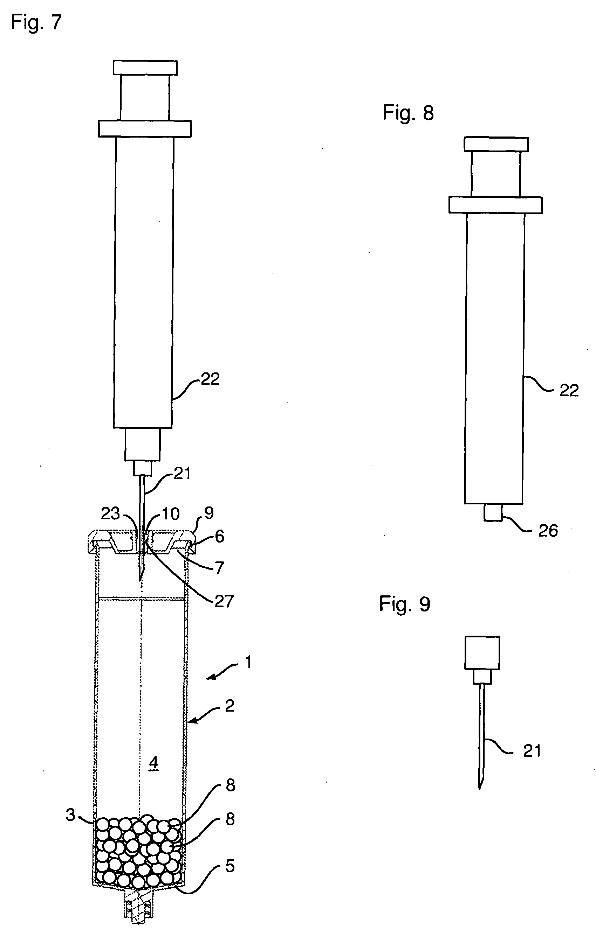

Um die gewünschte konditionierte Blutzusammensetzung in dem Behälter 1 herstellen zu können, wird z.B. mittels einer Einmalspritze 22 mit einer Einmalkanüle 21 einem Patienten (Mensch oder Tier) entnommenes Blut in den Behälter 1 eingefüllt (Fig. 7). Dazu wird die Einmalkanüle 21 durch die Einfüllöffnung 10 in den Hohlraum 4 eingeführt und dann das entnommene Blut aus der Einmalspritze 22 in den Hohlraum 4 eingefüllt.

Da der Hauptkörper 2 als starrer bzw. steifer Körper ausgebildet ist, sind die Abmessungen der Einfüllöffnung 10 bezogen auf die Einmalkanüle 21 so gewählt, daß zwischen der in der Einfüllöffnung 10 positionierten Einmalkanüle 21 und der Innenseite 27 (Fig. 4) der Einfüllöffnung 10 noch ein Entlüftungsspalt 23 (Fig. 7) vorgesehen ist, so daß Luft aus dem Hohlraum 4 beim Einfüllen des Blutes durch den Entlüftungsspalt 23 austreten kann. Die Einmalkanüle 21 füllt die Einfüllöffnung 10 also nicht vollständig aus, so daß aufgrund des Entlüftungsspaltes 23 eine Belüftung vorliegt und somit ohne Probleme Blut in den Hohlraum 4 eingefüllt werden kann.

Nach dem Einfüllen des Blutes wird die Einmalkanüle 21 aus der Einfüllöffnung 10 herausgezogen. Danach kann die Einfüllöffnung 10 mittels des Verschlußstopfens 13 (Fig. 6) verschlossen werden. Der Verschlußstopfen 13 ist auf den Luer Lock Anschluß 11 abgestimmt und weist ein auf das Außengewinde 12 angepaßtes Innengewinde 24 sowie einen Luer-Konus 25 zur Abdichtung der Einfüllöffnung 10 auf.

Der ggf. verschlossene Behälter 1 kann dann beispielsweise 10 Minuten bei 4.500 Umdrehungen/Minute zentrifugiert werden. Dies wird bevorzugt bei Raumtemperatur durchgeführt. Zusätzlich kann, muß aber nicht, eine Lagerung bei Raumtemperatur oder auch bei einer höheren Temperatur von bis zu 40°C und insbesondere bis zu 37°C des Behälters 1 durchgeführt werden. Diese Lagerung erfolgt bevorzugt vor dem Zentrifugieren.

Durch diese Schritte wird die gewünschte konditionierte Blutzusammensetzung hergestellt, in der beispielsweise IL-1Ra (Interleukin 1-Rezeptor-Antagonist) angereichert ist. Es können z.B. Konzentrationen von 150-750 pg/ml erzielt werden. Gleichzeitig ist die Konzentration von IL-1 β relativ gering, so daß das Verhältnis von IL-1β zu IL-1Ra von größer als 1:10, insbesondere größer als 1:100 und insbesondere größer als 1:250 erreicht wird. Es konnten Werte von 1:8800 erreicht werden.

Die so hergestellte konditionierte Blutzusammensetzung kann dann zu therapeutischen Zwecken eingesetzt werden. Insbesondere kann nach der Zentrifugierung das entstandene Serum aus dem Behälter 1 entnommen werden, indem der Verschlußstopfen 13 abgeschraubt wird (sofern er vorgesehen war) und mittels einer weiteren Einmalspritze 22 mit einer weiteren Einmalkanüle 21 das Serum entnommen wird. Auch hier ist wiederum der Außendurchmesser der weiteren Einmalkanüle 21 kleiner als der minimale Innendurchmesser der Einfüllöffnung 10, so daß auch beim Entnehmen des Serums der Entlüftungsspalt 23 und somit eine Entlüftung gegeben ist, wodurch ohne Probleme das Serum aus dem Behälter 1 entnommen werden kann. Das so gewonnene Serum kann dann in bekannter Art und Weise dem Patienten appliziert werden. Insbesondere kann es in das zu behandelnde Gelenk, im Bereich der zu behandelnden Nervenwurzel oder an sonstiger Stelle injiziert werden.

Der erfindungsgemäße Behälter 1 kann nicht nur alleine bereitgestellt werden. Insbesondere kann ein System zur Herstellung einer konditionierten Blutzusammensetzung bereitgestellt werden, das den Behälter 1, die Einmalspritze 22 (Fig. 8), die Einmalkanüle 21 (Fig. 9) und gegebenenfalls den Verschlußstopfen 13 (Fig. 6) aufweist. Bevorzugt sind alle Elemente des Systems einzeln steril verpackt. Natürlich kann das System noch weitere Elemente aufweisen, insbesondere können mehr als eine Einmalkanüle 21 vorgesehen sein. So kann beispielsweise die erste Einmalkanüle in Verbindung mit der Einmalspritze 22 verwendet werden, um das Blut dem Patienten zu entnehmen. Nach der Blutentnahme kann die benutzte Einmalkanüle 21 entsorgt und eine weitere Einmalkanüle 21 mit der Einmalspritze 22 verbunden werden und erst danach das entnommene Blut in den Behälter 1 eingefüllt werden. Damit kann z.B. verhindert werden, daß eine Kontamination der ersten Einmalkanüle 21 bei der Blutentnahme (z.B. durch die Einstichstelle) beim Umfüllen in den Hauptkörper 2 auf das Blut gegebenenfalls übertragen wird.

Natürlich ist es auch möglich, beim Einfüllen des Blutes von der Einmalspritze 22 in den Hauptkörper 2 die Einmalkanüle 21 vollständig wegzulassen. In diesem Fall müssen lediglich die Abmessungen der Einfüllöffnung 10 so gewählt sein, daß beim Einführen des vorderen Endes 26 der Einmalspritze 22 (Fig. 8) in die Einfüllöffnung 10 noch der gewünschte Entlüftungsspalt 23 vorliegt.

## Patentansprüche

1. Behälter für die Herstellung einer konditionierten Blutzusammensetzung, die insbesondere IL-1 Ra enthält, mit
einer einen Hohlraum (4) begrenzenden und eine Öffnung (7) aufweisende Wandung (3),
einer Mehrzahl von Teilchen (8), die in den Hohlraum (4) des Behälters (1) eingefüllt sind, und einer die Öffnung (7) verschließenden Verschlußkappe (9), die eine Einfüllöffnung (10) aufweist, in der ein Abgabeende (21, 26) einer Blut enthaltenden Spritze (22) positionierbar ist, um das Blut.der Spritze (22) in den Hohlraum (4) des Behälters (1) einzufüllen,
**dadurch gekennzeichnet, dass**
die Abmessungen der Einfüllöffnung (10) so gewählt sind, dass die Teilchen (8) zu groß für die Einfüllöffnung (10) sind und somit nicht durch sie hindurchpassen und dass, wenn das Abgabeende (21, 26) der Spritze (22) zum Einfüllen des Blutes in der Einfüllöffnung (10) positioniert ist, ein Entlüftungsspalt (23) zwischen dem Abgabeende (21, 26) und der Innenseite (27) der Einfüllöffnung (10) vorhanden ist.

2. Behälter nach Anspruch 1, **dadurch gekennzeichnet, dass** die Wandung (3) als starre Wandung ausgebildet ist.

3. Behälter nach Anspruch 1 oder 2, **dadurch gekennzeichnet, dass** die Verschlußkappe (9) die Öffnung (7) so verschließt, dass die Verschlußkappe (9) nicht zerstörungsfrei entfernbar ist.

4. Behälter nach einem der obigen Ansprüche, **dadurch gekennzeichnet, dass** die Verschlußkappe (9) mittels einer Rastverbindung mit der Wandung (3) verbunden ist.

5. Behälter nach einem der obigen Ansprüche, **dadurch gekennzeichnet, dass** die Wandung (3) und die Verschlußkappe (9) aus dem gleichen Material hergestellt sind.

6. Behälter nach einem der obigen Ansprüche, **dadurch gekennzeichnet, dass** die Einfüllöffnung (10) Bestandteil eines Luer Lock Anschlusses (11) ist.

7. Behälter nach einem der obigen Ansprüche, **dadurch gekennzeichnet, dass** die Teilchen (8) im wesentlichen kugelförmig ausgebildet sind.

8. Behälter nach einem der obigen Ansprüche, **dadurch gekennzeichnet, dass** die Teilchen (8) aus Glas hergestellt sind.

9. Behälter nach einem der obigen Ansprüche, **dadurch gekennzeichnet, dass** die Wandung (3) nur die eine Öffnung (7) aufweist.

10. Behälter nach einem der obigen Ansprüche, **dadurch gekennzeichnet, dass** die Einfüllöffnung (10) als offenes Durchgangsloch ausgebildet ist.

11. Behälter nach einem der obigen Ansprüche, **dadurch gekennzeichnet, dass** der Behälter bis auf die Einfüllöffnung (10) frei ist von weiteren Be- bzw. Entlüftungselementen.

12. Behälter nach einem der obigen Ansprüche, **dadurch gekennzeichnet, dass** Behälter aufgrund der Einfüllöffnung (10) für das einzufüllende Blut offen und für die Teilchen (8) geschlossen ist.

13. Behälter nach einem der obigen Ansprüche, **dadurch gekennzeichnet, dass** das Volumen des Hohlraums außer mit den eingefüllten Teilchen und dem einzufüllenden Blut nicht veränderbar ist.

14. System zur Herstellung einer konditionierten Blutzusammensetzung, die insbesondere IL-1Ra enthält, mit
einer ein Abgabeende (21, 26) aufweisenden Spritze (22),
**gekennzeichnet durch** einen Behälter (1) nach einem der obigen Ansprüche,
wobei der Entlüftungsspalt (23) zwischen dem Abgabeende (21, 26) und der Innseite (27) der Einfüllöffnung (10) vorhanden ist, wenn das Abgabeende (21, 26) der Spritze (22) zum Einfüllen von in der Spritze (22) enthaltenem Blut in der Einfüllöffnung (10) positioniert ist.

15. Verfahren zur Herstellung einer konditionierten Blutzusammensetzung, die insbesondere IL-1Ra enthält, **dadurch gekennzeichnet, dass**
das Abgabeende (21, 26) einer Spritze (22), die Blut enthält, in der Einfüllöffnung (10) eines Behälters (1) nach einem der Ansprüche 1 bis 13 so positioniert wird, dass der Entlüftungsspalt (23) vorhanden ist, dann das Blut von der Spritze (22) in den Hohlraum (4) des Behälters (1) eingefüllt wird,
und bei dem der Behälter mit dem Blut zentrifugiert wird, um die konditionierte Blutzusammensetzung herzustellen.

## Claims

1. Container for the production of a conditioned blood compound which comprises IL-1Ra, in particular, said container comprising
a wall (3) delimiting a cavity (4) and having an opening (7),
a plurality of particles (8) which are filled into the cavity (4) of the container (1), and a closure cap (9) closing the opening (7) and having a filling opening (10), in which a discharge end (21, 26) of a blood-containing syringe (22) can be positioned to fill the blood of the syringe (22) into the cavity (4) of the container (1),
**characterized in that**,
the dimensions of the filling opening (10) are adapted such that the particles (8) are too large for the filling opening (10) and therefore cannot escape through it, and, when the discharge end (21, 26) of the syringe (22) is positioned in the filling opening (10) to fill in blood, a vent gap (23) is provided between the discharge end (21, 26) and the inside (27) of the filling opening (10).

2. Container according to claim 1, wherein the wall (3) is formed as a rigid wall.

3. Container according to claim 1 or 2, wherein the closure cap (9) closes the opening (7) so that the closure cap (9) cannot be removed without causing destruction.

4. Container according to one of the above claims, wherein the closure cap (9) is connected to the wall (3) by way of a snap connection.

5. Container according to one of the above claims, wherein the wall (3) and the closure cap (9) are made of the same material.

6. Container according to one of the above claims, wherein the filling opening (10) is a component of a Luer Lock connection (11).

7. Container according to one of the above claims, wherein the particles (8) substantially comprise a spherical form.

8. Container according to one of the above claims, wherein the particles (8) are made of glass.

9. Container according to one of the above claims, wherein the wall (3) only has said one opening (7).

10. Container according to one of the above claims, wherein the filling opening (7) is formed as an open through-hole.

11. Container according to one of the above claims, wherein the container is free of further ventilation elements apart from the filling hole (10).

12. Container according to one of the above claims, wherein the container is open to the blood which shall be filled in but closed to the particles (8) due to the filling opening (10).

13. Container according to one of the above claims, wherein the volume of the cavity cannot be changed apart from the filled in particles and the blood which shall be filled in.

14. System for the production of a conditioned blood compound which comprises IL-1 Ra, in particular, said system comprising
a syringe (22) having a discharge end (21, 26),
**characterized by** a container (1) according to one of the above claims,
wherein the vent gap (23) is provided between the discharge end (21, 26) and the inside of the filling opening (10) when the discharge end (21, 26) of the syringe (22) is positioned for filling the blood, contained in the syringe (22), in the filling opening (10).

15. Method for the production of a conditioned blood compound which comprises IL-1 Ra, in particular, **characterized in that**,
the discharge end (21, 26) of a blood-containing syringe (22) is positioned in the filling opening (10) of a container (1) according to one of the claims 1 to 13, such that the vent gap (23) is provided, the blood is filled from the syringe (22) into the cavity (4) of the container (1) then, and centrifuging the container with the blood to produce the conditioned blood composition.

## Revendications

1. Récipient pour la fabrication d'une composition sanguine conditionnée, qui contient en particulier IL-1Ra, avec
une paroi (3) limitant une cavité (4) et présentant une ouverture (7), une pluralité de particules (8) qui sont introduites dans la cavité (4) du récipient (1), et un capuchon de fermeture (9) fermant l'ouverture (7) qui présente une ouverture de remplissage (10) dans laquelle une extrémité de distribution (21, 26) d'une seringue (22) contenant du sang peut être positionnée pour introduire le sang de la seringue (22) dans le cavité (4) du récipient (1), **caractérisé en ce que**
les dimensions de l'ouverture de remplissage (10) sont choisies de telle sorte que les particules (8) sont trop grandes pour l'ouverture de remplissage (10) et ne peuvent pas la franchir, et **en ce que**, quand l'extrémité de distribution (21, 26) est positionnée dans l'ouverture de remplissage (10) pour l'introduction du sang, il y a un interstice de désaération (23) entre l'extrémité de distribution (21, 26) et le côté intérieur de l'ouverture de remplissage (10).

2. Récipient selon la revendication 1, **caractérisé en ce que** la paroi (3) est constituée en tant que paroi rigide.

3. Récipient selon la revendication 1 ou 2, **caractérisé en ce que** le capuchon de fermeture (9) ferme l'ouverture (7) de telle sorte que le capuchon de fermeture (9) ne peut pas être enlevé de façon non destructrice.

4. Récipient selon l'une des revendications ci-dessus, **caractérisé en ce que** le capuchon de fermeture (9) est raccordé à la paroi (3) au moyen d'un raccordement par encliquetage.

5. Récipient selon l'une des revendications ci-dessus, **caractérisé en ce que** la paroi (3) et le capuchon de fermeture (9) sont fabriqués avec le même matériau.

6. Récipient selon l'une des revendications ci-dessus, **caractérisé en ce que** l'ouverture de remplissage (10) est un composant d'un raccord Luer Lock (11).

7. Récipient selon l'une des revendications ci-dessus, **caractérisé en ce que** les particules (8) sont constituées essentiellement sous forme sphérique.

8. Récipient selon l'une des revendications ci-dessus, **caractérisé en ce que** les particules (8) sont fabriquées en verre.

9. Récipient selon l'une des revendications ci-dessus, **caractérisé en ce que** la paroi (3) ne présente qu'une seule ouverture (7).

10. Récipient selon l'une des revendications ci-dessus, **caractérisé en ce que** l'ouverture de remplissage (10) est constituée en tant que trou traversant ouvert.

11. Récipient selon l'une des revendications ci-dessus, **caractérisé en ce que**, à part l'ouverture de remplissage (10), le récipient est exempt d'autres éléments d'aération ou respectivement de désaération.

12. Récipient selon l'une des revendications ci-dessus, **caractérisé en ce que** le récipient, du fait de l'ouverture de remplissage (10), est ouvert pour le sang à introduire et fermé pour les particules (8).

13. Récipient selon l'une des revendications ci-dessus, **caractérisé en ce que** le volume de la cavité n'est pas modifiable excepté avec les particules introduites et le sang à introduire.

14. Système destiné à la fabrication d'une composition sanguine conditionnée qui contient en particulier IL-1Ra, avec
une seringue (22) présentant une extrémité de distribution (21, 26), **caractérisé par** un récipient (1) selon l'une des revendications ci-dessus, l'interstice de désaération (23) étant présent entre l'extrémité de distribution (21, 26) et le côté intérieur (27) de l'ouverture de remplissage (10) quand l'extrémité de distribution (21, 26) de la seringue (22) est positionnée (10) dans l'ouverture de remplissage pour l'introduction du sang contenu dans la seringue (22).

15. Procédé destiné à la fabrication d'une composition sanguine conditionnée qui contient en particulier IL-1Ra, **caractérisé en ce que** l'extrémité de distribution (21, 26) d'une seringue (22) qui contient du sang est positionnée dans l'ouverture de remplissage (10) d'un récipient (1) selon l'une des revendications 1 à 13 de telle sorte que l'interstice de désaération (23) est présent, puis le sang de la seringue (22) est introduit dans la cavité (4) du récipient (1),
et dans lequel le récipient avec le sang est centrifugé pour fabriquer la composition sanguine conditionnée.
